# EUROPEAN PATENT APPLICATION

(11) **EP 4 098 195 A1**
(43) Date of publication of application: **07.12.2022**
(21) Application number: 22175401.3
(22) Date of filing: 25.05.2022
(51) Int. Cl.: A61B 5/16, A61B 5/347, A61B 5/374, A61B 5/00, G08B 21/02, G08B 21/06

(54) **PHYSIOLOGICAL AND BEHAVIOURAL METHODS TO ASSESS PILOT READINESS**

(30) Priority: 04.06.2021 US 202163196784 P; 17.05.2022 US 202217746539
(71) Applicant: Rockwell Collins, Inc., Cedar Rapids, IA 52498 (US)
(72) Inventor: WU, Peggy, East Hartford, CT (US); RAO, Arjun Harsha, Marion, IA (US); MATESSA, Michael P., Ben Lomond, CA (US); WITTKOP, Timothy J., Marion, IA (US); GEORGE, Christopher L., Winchester, VA (US); JOHNSON, Wade T., Cedar Rapids, IA (US)
(74) Representative: Dehns

(57) **Abstract**

A system and method for automatically assessing pilot readiness via a plurality of biometric sensors (108, 110) includes continuously receiving biometric data including vision-based data; the biometric vision-based data is compared to a task specific set of movements and facial expressions as defined by known anchor points. A deviation is calculated based on the vision-based data and task specific set of movements and expressions, and the deviation is compared to an acceptable threshold for pilot readiness. Other biometric data may be included to refine the readiness assessment.

## Description

### PRIORITY

The present application claims the benefit under 35 U.S.C. § 119(e) of U.S. Provisional App. No. 63/196,784 (filed June 4, 2021).

### BACKGROUND

Pilot readiness for flight is guided by written policies and regulations, such as the limitations of flight hours, minimum rest hours immediately before duty, restrictions of alcohol use, and the availability of adequate sleep facilities. For single-pilot operations, the FAA provides guidelines such as the I'm SAFE checklist. Real-time evaluation of pilot readiness is primarily performed as self-assessment, or informally by fellow crew members. In either case, personal judgements are used to make the assessment, and they are primarily based on subjective observations that are subject to bias and human error, and place an additional burden on the crew.

### BRIEF DESCRIPTION OF THE DRAWINGS

The numerous advantages of the embodiments of the inventive concepts disclosed herein may be better understood by those skilled in the art by reference to the accompanying figures in which:
FIG. 1 shows a block diagram of a system according to an exemplary embodiment;
FIG. 2 shows a flowchart of a method according to an exemplary embodiment;
FIG. 3 shows a block diagram of a machine learning according to an exemplary embodiment;

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

Broadly, embodiments of the inventive concepts disclosed herein are directed to a system and method for automatically assessing pilot readiness via a plurality of biometric sensors. A processor continuously receives biometric data including vision-based data; the biometric vision-based data is compared to a task specific set of movements and facial expressions as defined by known anchor points. A deviation is calculated based on the vision-based data and task specific set of movements and expressions, and the deviation is compared to an acceptable threshold for pilot readiness. Other biometric data may be included to refine the readiness assessment.

Embodiments of the present disclosure may be more fully understood with reference to U.S. Patent App. No. 63/196,765 ("Cognitive Battery for Return to Service") (filed June 4, 2021) and U.S. Patent App. No. 63/196,798 ("Embedding Sensors in an Aircraft Control") (filed June 4, 2021).

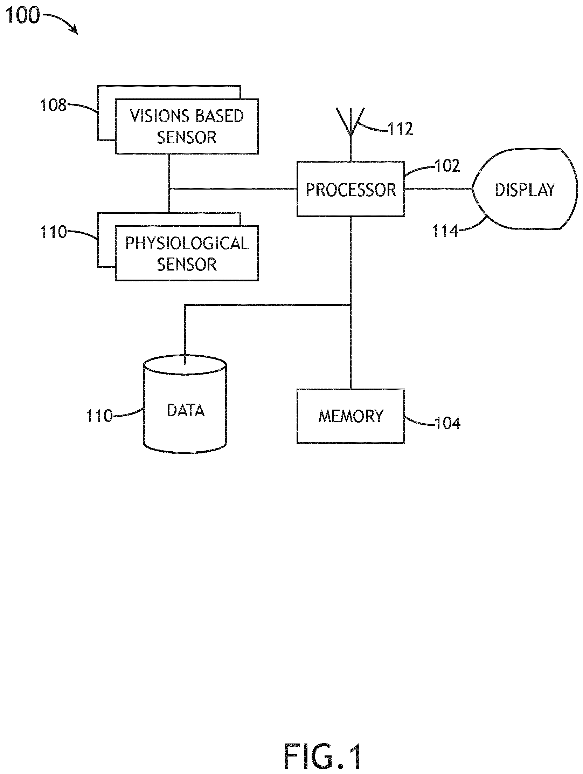

Referring to FIG. 1, a block diagram of a system according to an exemplary embodiment is shown. The system 100 includes a processor 102, memory 104 in data communication with the processor 102 for storing processor executable code, a data storage device 106 in data communication with the processor 102 for storing biometric data and task specific biometric profiles corresponding to idealized or otherwise acceptable biometric data corresponding to a state of readiness during a specific task, and one or more biometric data gathering devices 108, 110 for receiving biometric data.

In at least one embodiment, the one or more biometric data gathering devices 108, 110 include one or more vision-based sensors 108; for example, head or eye tracking cameras to record a pattern of head or eye movement of a pilot. In at least one embodiment, the one or more biometric data gathering devices 108, 110 include physiological sensors 110; for example, an electroencephalograph (EEG), an electrocardiograph (ECG or EKG), galvanic skin response sensor (GSR), pulse sensor, or other such biometric data sensing device. Physiological signals, such as EEG, ECG, and GSR, respond strongly to changes in the limbic system effected by a person's attention and cognitive engagement.

In at least one embodiment, the processor executable code configures the processor 102 to receive biometric data from the one or more vision-based sensors 108 and/or physiological sensors 110, and continuously log the biometric data in the data storage element 106. The processor executable code configures the processor 102 to analyze the biometric data with reference to a set of movements defined by identifiable anchor points on the pilot (hands, shoulders, eyes, etc., according to object recognition algorithms known in the art). Flight phases may be defined by a set of movements and expressions indicating readiness (desirable scan pattern, movements corresponding to the execution of a checklist, etc.); where the pilot's actual movements and expressions deviate from the defined set of movements and expressions, the pilot's readiness may be in question.

In at least one embedment, pilot readiness may be characterized with respect to performance impairment and incapacitation. Performance impairment indicates a pilot's continued ability to perform limited flight duties with degraded performance but continued control of the aircraft; impairment conditions include moderate gastrointestinal distress or pain, presyncope or light headedness, workload saturation, etc. Incapacitation, by comparison, indicates an inability to perform any flight duties for at least ten minutes; incapacitation conditions may include acute gastrointestinal distress or pain, syncope or loss of consciousness from neurological, cardiac, environmental, or urological conditions, or laser strikes, etc.

In at least one embodiment, the system 100 includes a communication device 112 in data communication with the processor 102 to provide pilot readiness data to a separate system such as a flight control or ground control system.

In at least one embodiment, the processor 102 may be configured to instantiate an artificial intelligence system such as a trained neural network to evaluate the biometric data for pilot readiness.

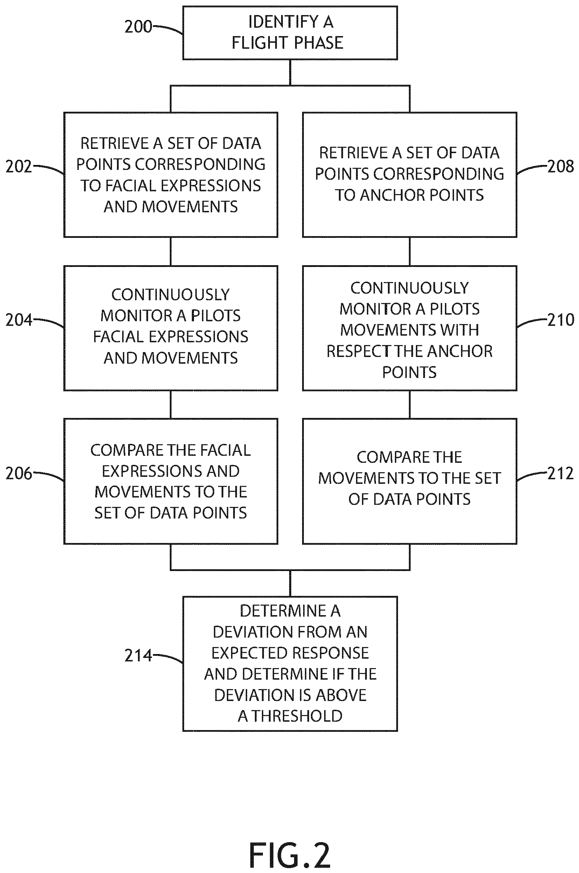

Referring to FIG. 2, a flowchart of a method according to an exemplary embodiment is shown. A processor receives biometric data, including vison-based biometric data, identifies 200 a current flight phase, including preflight phases such as executing a preflight checklist. The processor then retrieves 202 a set of data points corresponding to expected facial expressions, including a head pose, of a pilot during that flight phase. Such data points may be predefined according to recognized best practices, or they may be defined by a machine learning algorithm utilizing recorded sets of training data. The processor then continuously monitors 204 and analyzes a data stream of vision-based biometric data according to known eye-tracking and head-pose estimation algorithms. The identified eye movements and head-pose estimations are compared 206 to a set of data points corresponding to expected facial expressions and head-pose movements to determine 214 a deviation. The deviation may correspond to a simple variance between the expected facial expressions and head-pose movements and the actual identified eye movements and head-pose estimations over time, or a weighted function defined by a historical training data.

In at least one embodiment, the processor may also retrieve 208 anchor point locations corresponding to desirable locations of a pilot's physiology (hands, shoulders, etc.) during the flight phase. The processor then continuously monitors 210 and analyzes the data stream according to known object tracking algorithms. The identified physiology locations are compared 212 to the retrieved anchor points. The deviation may then include the comparison of actual locations to expected anchor points.

In at least one embodiment, comparison 212 of movement with respect to anchor points comprises a behavioral analysis, mapping the pilot's movements to expected behaviors during the flight phase, and with respect to eye movements and head-pose movements. In at least one embodiment, data from the video stream may include audio data that may further refine the readiness analysis, for example by analyzing cadence, tone, volume, and enunciation for markers of fatigue, distraction, irritability, etc.

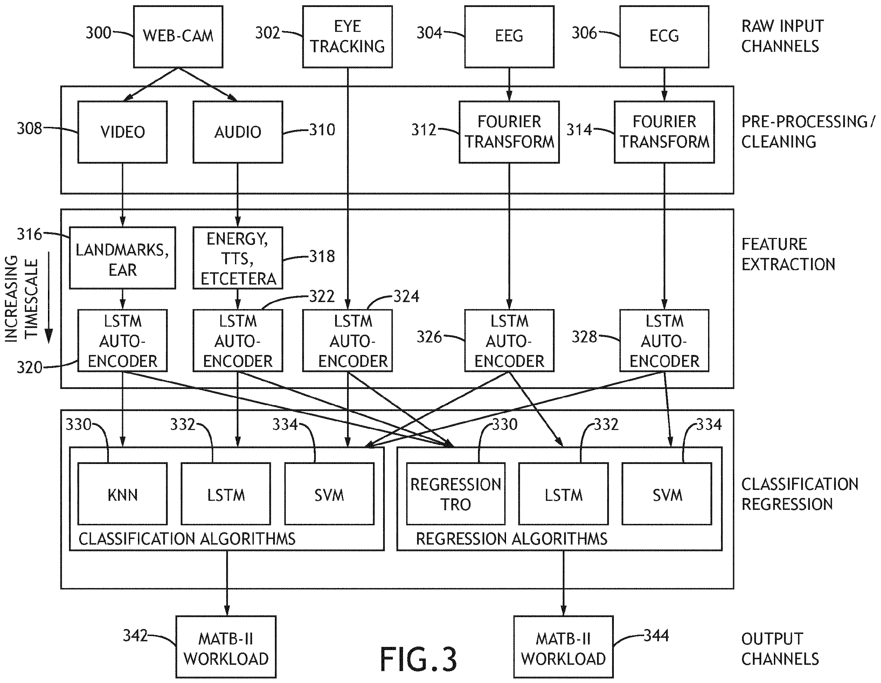

Referring to FIG. 3, a block diagram of a machine learning method according to an exemplary embodiment is shown. During a readiness analysis, a plurality of biometric inputs 300, 302, 304, 306 such as a camera 300 mounted with a field-of-view of the pilot, an eye tracking camera 302, an EEG sensor 304, and an ECG sensor 306 provide streaming biometric data. Such data may be supplied continuously or periodically as dictated by the workload of the flight phase and bandwidth of the system. During a preprocessing step, camera data may be bifurcated into a video stream 308 and an audio stream 310 for separate processing; furthermore, EEG data and ECG data may be processed, for example via frequency decomposition such as Fourier transformation.

After pre-processing, relevant features of the data streams are extracted. For example, the video stream 308 may be analyzed for expected physical landmarks 316 while the audio stream 310 may be analyzed via a test-to-speech algorithm 318. In at least one embodiment, each pre-processed data stream 308, 310, 312, 314 may be processed via an autoencoder 320, 322, 324, 326, 328 such as an LSTM autoencoder for classification and analysis by trained classification algorithms 330, 332, 334 and regression algorithms 336, 338, 340. In at least one embodiment, classification algorithms may include a K-Nearest Neighbor algorithm 330, a long short-term memory algorithm 332, a support vector machine 334, etc. In at least one embodiment, the regression algorithms may include a regression tree 336, a long short-term memory algorithm, a support vector machine 340, etc.

In at least one embodiment, the classification algorithms 330, 332, 334 are trained to classify a pilot's workload 342 or physiological response to a workload; likewise the regression algorithms 336, 338, 240 are trained to characterize the pilots performance 344. Together these metrics may be utilized to assess the pilot's readiness with respect to a current flight phase.

In at least one embodiment, the system may be trained via reference to an EEG sensor 304 and an ECG sensor 306 which are highly sensitive to changes in a pilot's limbic system; but the actual classification is performed with respect to the camera 300 and eye-tracking camera 302.

Embodiments of the present disclosure aggregate facial expression recognition and pose to objectively evaluate a pilot's readiness for duty. This method is based on computer vision and non-intrusive. It can be used preflight while the pilot is performing preflight checklist duties, as well as in the air such as during long-haul flights. Camera sensor pointed at the pilot first locate the pilot's face and body anchor points. Deviations of the anchor points from their nominal position are calculated to arrive at a measure of muscle movement, providing time series data that can then be used as an objective assay to pilot fitness for duty. These methods can evaluate whether those facial and pose changes are commonly associated with manifestations of pilot states that can potentially impair performance, such as illness, fatigue, alcohol use, or stress.

This method provides an objective, impartial evaluation of pilot readiness throughout different phases of flight. It can be used to alert the pilot as well as supporting personnel of potential adverse events. Embodiments are useful for pilots in military and commercial settings, and particularly in single pilot operations or emergency medical air services.

It is believed that the inventive concepts disclosed herein and many of their attendant advantages will be understood by the foregoing description of embodiments of the inventive concepts disclosed, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The form herein before described being merely an explanatory embodiment thereof, it is the intention of the following claims to encompass and include such changes. Furthermore, any of the features disclosed in relation to any of the individual embodiments may be incorporated into any other embodiment.

## Claims

1. A computer apparatus comprising at least one processor (102) in data communication with a plurality of cameras and a memory storing processor executable code for configuring the at least one processor to:
identify a current flight phase;
retrieve a set of expected data corresponding to expected positions of physiological landmarks during the flight phase;
receive an image stream from each of the plurality of cameras;
identify one or more physiological landmarks of a pilot in the image streams;
continuously analyze a position of the physiological landmarks with respect to the set of expected data to determine an actual deviation; and
determine if the actual deviation is within an acceptable threshold of readiness for duty.

2. The computer apparatus of Claim 1, wherein:
the at least one processor is further configured to:
receive a data stream from an EEG sensor;
receive a data stream from and ECG sensor; and
determine a cognitive performance metric based on the EEG sensor data stream and the ECG sensor data stream; and
the actual deviation is further **characterized by** the cognitive performance metric.

3. The computer apparatus of Claim 2, wherein the at least one processor is further configured to:
pre-process the EEG sensor data stream and ECG sensor data stream via a frequency decomposition algorithm; and
pre-process at least one image stream into a video stream and an audio stream.

4. The computer apparatus of Claim 3, wherein the at least one processor is further configured to identify the physiological landmarks via one or more of a K-Nearest Neighbor classification algorithm, a long short-term memory classification algorithm, and a support vector machine; and/or wherein the at least one processor is further configured to extract audio features via a text-to-speech algorithm.

5. The computer apparatus of any preceding Claim, wherein at least one video stream comprises an eye-tracking video stream.

6. The computer apparatus of any preceding Claim, wherein the at least one processor is further configured to:
determine if a deviation is associated with fatigue, illness, or stress; and
implement a warning procedure that the pilot is not within a threshold of readiness.

7. A system for characterizing a pilot's readiness for duty comprising:
a plurality of cameras (300);
an EEG sensor (304);
an ECG sensor (306); and
at least one processor in data communication with the plurality of cameras, the EEG sensor, the ECG sensor, and a memory storing processor executable code for configuring the at least one processor to:
identify a current flight phase;
retrieve a set of expected data corresponding to expected positions of physiological landmarks during the flight phase;
receive a data stream from an EEG sensor;
receive a data stream from and ECG sensor;
determine a cognitive performance metric based on the EEG sensor data stream and the ECG sensor data stream;
receive an image stream from each of the plurality of cameras;
identify one or more physiological landmarks of a pilot in the image streams;
continuously analyze a position of the physiological landmarks with respect to the set of expected data and the cognitive performance metric to determine an actual deviation; and
determine if the actual deviation is within an acceptable threshold of readiness for duty.

8. The system of Claim 7, wherein the at least one processor is further configured to:
pre-process the EEG sensor data stream and ECG sensor data stream via a frequency decomposition algorithm; and
pre-process at least one image stream into a video stream and an audio stream, and preferably wherein the at least one processor is further configured to identify the physiological landmarks via one or more of a K-Nearest Neighbor classification algorithm, a long short-term memory classification algorithm, and a support vector machine; and/or
wherein the at least one processor is further configured to extract audio features via a text-to-speech algorithm.

9. The system of Claim 7 or 8, wherein at least one video stream comprises an eye-tracking video stream.

10. The system of Claim 7, 8 or 9, wherein the at least one processor is further configured to:
determine if a deviation is associated with fatigue, illness, or stress; and
implement a warning procedure that the pilot is not within a threshold of readiness.

11. A method of characterizing a pilot's readiness for duty comprising:
identifying a current flight phase;
retrieving a set of expected data corresponding to expected positions of physiological landmarks during the flight phase;
receiving an image stream from each of a plurality of cameras;
identifying one or more physiological landmarks of a pilot in the image streams;
continuously analyzing a position of the physiological landmarks with respect to the set of expected data to determine an actual deviation; and
determining if the actual deviation is within an acceptable threshold of readiness for duty.

12. The method of Claim 11, further comprising:
receiving a data stream from an EEG sensor;
receiving a data stream from and ECG sensor; and
determining a cognitive performance metric based on the EEG sensor data stream and the ECG sensor data stream; and
wherein the actual deviation is further **characterized by** the cognitive performance metric.

13. The method of Claim 12, further comprising:
pre-processing the EEG sensor data stream and ECG sensor data stream via a frequency decomposition algorithm; and
pre-processing at least one image stream into a video stream and an audio stream, and preferably further comprising identifying the physiological landmarks via one or more of a K-Nearest Neighbor classification algorithm, a long short-term memory classification algorithm, and a support vector machine; and/or
further comprising extracting audio features via a text-to-speech algorithm.

14. The method of Claim 11 or 12, wherein at least one video stream comprises an eye-tracking video stream.

15. The method of Claim 11, 12 or 13, further comprising:
determining if a deviation is associated with fatigue, illness, or stress; and
implementing a warning procedure that the pilot is not within a threshold of readiness.
